(19) Europäisches Patentamt
European Patent Office
Office européen des brevets

(11) Publication number: **0 416 675 A1**

# (12) EUROPEAN PATENT APPLICATION

(21) Application number: **90202158.3**

(22) Date of filing: **09.08.90**

(51) Int. Cl.[5]: **C07D 487/04**, C08K 5/3415, C09B 57/00, //(C07D487/04, 209:00,209:00)

(30) Priority: **04.09.89 IT 2161489**

(43) Date of publication of application: **13.03.91 Bulletin 91/11**

(84) Designated Contracting States: **AT BE CH DE DK ES FR GB GR LI LU NL SE**

(71) Applicant: **ENICHEM SYNTHESIS S.p.A.**
**Via Ruggero Settimo 55**
**I-90139 Palermo(IT)**

(72) Inventor: **Cassar, Luigi**
**Via Europa 42**
**I-20097 San Donato Milanese, Milan(IT)**
Inventor: **Villanti, Alberto**
**Viale delle Rimembranze di Lambrate 9**
**I-20134 Milan(IT)**

(74) Representative: **Roggero, Sergio et al**
**Ing. Barzanò & Zanardo Milano S.p.A. Via Borgonuovo 10**
**I-20121 Milano(IT)**

## (54) High stability pigments belonging to the 1,4-diketone pyrrole-[3,4-C]-pyrroles and new pyrrole-[3,4-C]-pyrroles series, suitable for the purpose.

(57) A method is described for giving colour to organic materials and for the preparation of paints and varnishes of excellent stability.

This method is envisaged for the use of 1,4-diketone pyrrole-[3,4-c]-pyrroles, according to the general formula (I).

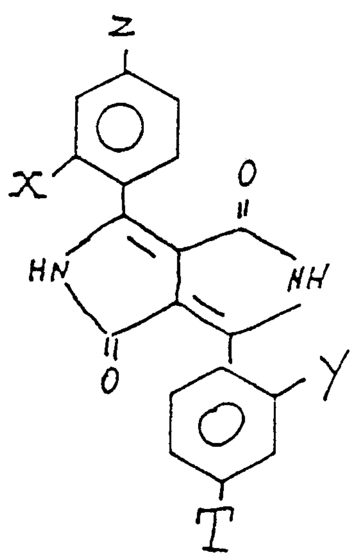

EP 0 416 675 A1

## HIGH STABILITY PIGMENTS BELONGING TO THE 1,4-DIKETONE PYRROLE-[3,4-C]-PYRROLES AND NEW PYRROLE-[3,4-C]-PYRROLES SERIES, SUITABLE FOR THE PURPOSE

This invention refers to pigments of exceptional stability for organic materials of high molecular weight, as well as for the preparation of paints and varnishes, according to the general formula (I)

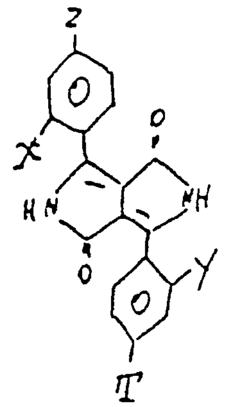

in which X, Y, Z and T have the following meanings.

It also refers to the procedure for the preparation of these compounds, as well as to new pyrrole-[3,4-c]-pyrroles which fall under the abovementioned general formula (I)

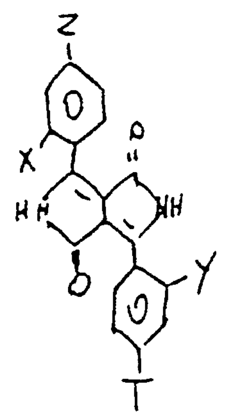

The 1,4-diketone-′3,6-diphenylpyrrole-[3,4-c]-pyrroles have been known for some time, being prepared for reaction between benzonitrile and ethylbromacetate as described in Tetrahedron Lett. 1974, 2549-52: the reaction allows use of a curing agent containing Zn and Cu, but gives only moderate yields.

Analogous compounds are also prepared with decidedly improved yields, by causing reaction of an ester of succinic acid with a suitable nitrile, as described in the United States patent no. 4,579,949.

This patent in fact describes a process for the preparation of 1,4-diketone pyrrole-[3,4-c]-pyrroles of formula

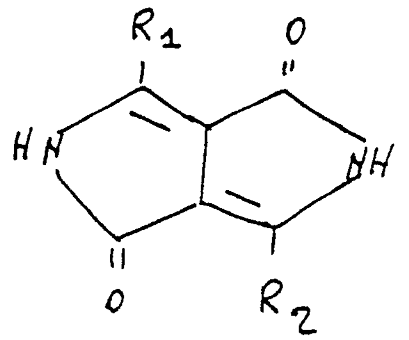

(II)

that is a process consisting of causing a reaction of a mol of a disuccinate with two mol of a nitrile selected from $R_1$-CN and $R_2$-CN: $R_1$ and $R_2$ are aromatic, isocyclic and heterocyclic radicals. The reaction is carried out at a temperature of between 60 and 140° C, in the presence of a strong base and the product thus obtained is subject to hydrolysis.

Returning to the groups substituting $R_1$ and $R_2$, they may be equal or different, simple or, on the other hand, substituted: when present, no substituting element is however provided for in the ortho position with respect to the radical connection (ortho, that is, with respect to the carbon atom linked to positions 3 and/or 6 of the double heterocyclic pyrrole-pyrrolic ring).

A diketone pyrrole of formula (II), with $R_1$ equal to phenyl and $R_2$ equal to 2-chlorophenyl, is obtainable according to example 10 of the United States patent no. 4,659,775, in which the reaction between 3-ethoxycarbonyl-2-phenyl-2-pyrrolin-5-one with 2-chlorobenzonitrile is described. It is example no. 10, the only case of the presence of an ortho substituting element, in one of the two groups in positions 2 and 6 of the pyrrole-pyrrolic cycle, the whole description of the patent in question making constant reference to the presence of possible substituting elements in the meta and para positions.

As mentioned during presentation of the formula (I) compounds, the derivates including the pyrrolic bicyclic structure may be used to give colouration to organic materials of high molecular weight, in hues which vary from yellow to red, and the abovementioned United States references do not hide the good results obtainable by means of use of 1,4-diketone pyrroles selected from among those of the (II) formula. These references are both patented in the United States, as stated, and are both in the Ciba-Geigy name; the same Ciba is owner of a fairly sizeable package of patent rights on the subject, with priority from '82 to '86, and it is surprising that all the relevant descriptions (excluding the abovementioned US 4,659,775) should define and exemplify structures of 1,4-diketone pyrrole pyrrolic[3,4-c] containing regroupings in 2 and 6, in which these regroupings, when constituted by cyclic radicals, are never ortho substituted with respect to the radical position.

Returning to US patent 4,379,949, it should also be stressed that, when aromatic nitriles are taken into consideration, in the reaction with the disuccinate, and the ring linked to the CN group contains substituting elements, the same are always found in meta or para position with respect to this group.

In fact, a structure in the (III) formula [Argew. Chem. Int. Ed. Engl. 28 (1989), no. 4, 478-480]

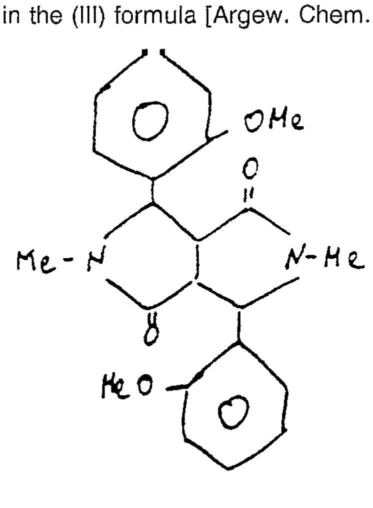

(III)

to which notable fluorescence is attributed, is well known. This compound is prepared by causing a reaction of 2-methoxybenzonitrile with di-isopropylsuccinate, subsequently causing alkylation of the product thus obtained.

The applicant has now found that a first object of this invention lies in the possibility to give mass colouration to organic materials of higher molecular weight, or prepare paints and varnishes, using derivatives of 1,4-diketone pyrrole -[3,4-c]pyrrolics, according to the subject of this invention, as in the following formula (I)

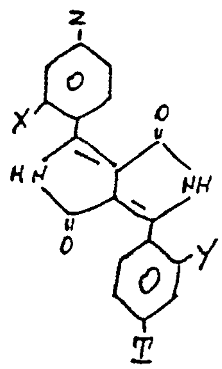

in which X and Y, equal or different from each other, are selected between

a) halogen atoms;

b) linear or branched alkylic groups, simple or substituting, containing up to five carbon atoms;

c) $-OR_3$ groups, in which $R_3$ may be an alkylic radical, either simple or substituted;

d) $-SR_3$ groups;

g) carboxylic groups;

h) ether groups;

i) amidic groups, in which, still, Z and T, either equal or different, may have one of the meanings attributed to X or Y, or be equal to H.

As already mentioned, pyrrolic derivatives belonging to the general formula (I) are useful pigments for organic materials of high molecular weight, as well as being intermediaries for the preparation of paints and varnishes.

In particular, and this is quite surprising, these derivatives have shown that they possess pigment qualities towards polymer-type organic materials which are far higher than those shown by similar products, para or meta substituted with respect to positions 3 and/or 6 of the double etherocyclic ring, which constitute the subject of the various references of the known part previously examined.

In fact, artificial ageing tests carried out on LD polyethylene plates (riblene 2100) in WOM atlas Ci 65 weatherometer, showed, after 1000 hours exposure, stability of products ortho substituted with respect to those shown by the para-chloro derivatives treated and exposed in the same way.

Similar results were obtained on PVC plates, prepared as described in example 6.

The compounds concerned in this invention are obtainable according to a procedure, also subject of the invention, which consists of causing the reaction of a benzonitrile, ortho substituted, with a di-alkylic ester of succinic acid; it is clear that the benzene ring of the nitrile may contain other substituting elements as well as those provided for in the structure of formula (I): the presence of these substituting elements is translated into a higher complexity of this same structure, without the final compounds departing from the spirit of this invention. The abovementioned reaction is carried out in a solvent preferably constituted by a tertiary alcohol in the presence of a base, at a temperature varying between 50 and 120 °C, and finally the product of condensation is hydrolysed at a temperature between 50 and 90 °C.

Should the benzonitrile be solid at ambient temperature and insoluble in the succinic ester, both can be dissolved in an inert organic solvent of low boiling point and thus drip this solution in 5-7 hours on the tertiary alkaline alcohol maintained at the reaction temperature, in a reactor equipped with a thermostat condenser at the right temperature for making the tertiary alcohol flow back and so that at the same time the low-boiling inert organic solvent, and the alcohol which frees itself from the succinic ester during reaction, can freely evaporate.

Having completed the addition the reaction is left to complete for a further 1-2hours and thus, having brought the temperature to 60-80 °C the mass of reaction hydrolyses by adding an organic acid, preferably acetic acid, which provokes the precipitation of the pigment.

This is finally gathered by filtration, washed and dried.

In certain cases, further purification may be necessary by means of washing with acetone in soxlet.

The pigments thus obtained are generally also equipped with fairly high covering power according to its

microcrystalline form, and can be incorporated in organic materials of high molecular weight, according to known techniques, giving the above materials an intense and glossy colouration, highly resistant to the action of humidity, heat and UV radiation, and certainly more resistant than similar colourations given by 1,4-diketone-3,6-diphenyl-pyrrole-[3,4-c]-pyrroles not ortho substituted in the phenols.

The abovementioned pigments are also extremely suitable for preparing high quality paints, varnishes and printing inks.

**EXAMPLE NO. 1**

Part A

A large ball of 500 ml is equipped with a condenser connected with a thermostat and a high velocity compressed air mixer which activates a turbine for dispersion of sodium.

6.9 g of sodium chips and 120 ml of tri-amylic alcohol are charged in nitrogen atmosphere.

It is heated to reflux and is quickly mixed (2000 rpm) until the sodium is completely dissolved.

Finally the temperature of the condenser is brought to 85° C.

Part B

20 g of di-isopropyl succinate and 22.7 g of 2-Cl-bennitrile are dissolved in ethylic ether.

The solution is dripped for 7 hours on the solution of tri-amylate of sodium kept at reflux and thus the reaction is left to complete for 2 hours. It is cooled to 60° C and the reaction mass diluted with 140 ml of methanol. Thus in 1 hour a solution consisting of 20 ml of glacial acetic acid is added and 15 ml of methanol, thus provoking the precipitation of the pigment.

It is separated by warm filtration and subsequently washed with 250 ml of boiling methanol and 200 ml of boiling water.

The filtered matter is dried under vacuum at 90° C.

11.4 g of orange pigment are obtained, equal to 32% of the theoretic pigment of formula 1.

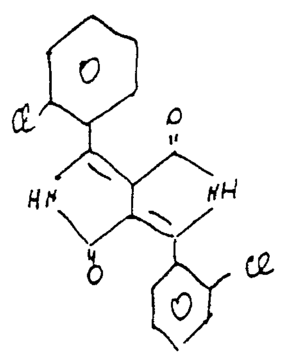

(1)

**EXAMPLE NO. 2**

In a tri-amylic sodium solution prepared as shown in example no. 1, part A, a mixture of 20 g of di-isopropyl succinate and 23.2 g of di-o-tolunitriles dripped for 6 hours.

Operating as described in part B of example 1, 2.03 g of gloss yellow pigment are obtained, equal to 6.5%of the theoretical pigment of formula 2

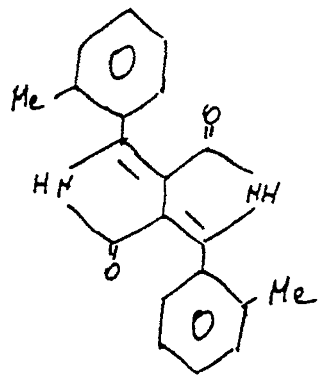

(2)

**EXAMPLE NO. 3**

Proceeding as described in example 2 and using 26.4 g of 2-methoxy benzonitrile, 1.98 g of strawberry red colour are obtained, equal to 6% of the theoretic pigment of formula 3:

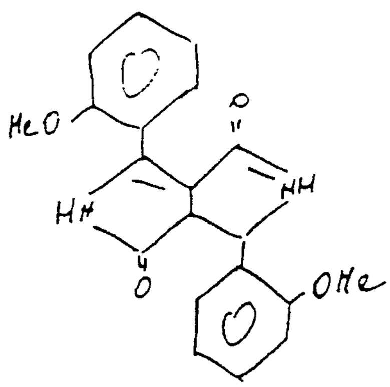

(3)

**EXAMPLE NO. 4**

Proceeding as in example 1 and using 34 g of 2.4 dichlorobenzonitrile, 19.5 g of violet-red pigment are obtained, equal to 23% of the theoretic pigment of formula 4:

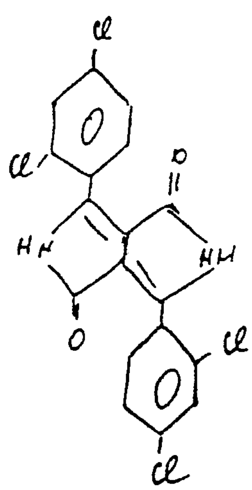

(4)

**EXAMPLE NO. 5**

Plates of polyethylene LD.

1 part of the formula 2 pigment (see example 2), 0.07 parts of ANOX 20 and 100 parts of polyethylene in granules were processed in the plastification chamber of a Brabender plastograph at 135° C for 10′.

1 mm plates of printing thickness and compressed in a Carver press at 190° C for 4′, were obtained from the mixture.

The samples thus obtained featured a very intense gloss yellow colour which remains substantially unchanged after 1000 hours of WOM ageing.

**EXAMPLE NO. 6**

PVC plates

0.5 parts of formula 1 pigment (see example 1), 100 g of PVC, 40 g of dioctyl phthalate, 2.5 g of dibutyl maleate tin were processed in a roller mixer for 15′ at 160° C, and transformed to 0.5 mm thick sheets.

The pigment gives the film thus obtained a bright yellow-orange colour, substantially unchanged after 1000 hours in WOM.

**EXAMPLE NO. 7**

Melamine acrylic lacquer

5 g of formula 2 pigment (see example 2) were added to 95 g of lacquer prepared thus: 20 parts of solution of a solution of 60% acrylic resin in xylol, 8 parts of a solution of 55% melamine-formaldehyde resin butanol, 2.5 parts of glycol acetate, 0.5 parts of silicone A oil (1% in xylol) and 16 parts of xylene are mixed in a mixer until a homogeneous light-coloured lacquer solution is obtained.

Having added the pigment to the lacquer, the whole amount is placed in a spherical mill for 60 hours.

A high gloss yellow lacquer is obtained, which, when applied with the usual methods on a metal plate, is oven-dried at 120° C for 20′, giving the plate a very bright yellow colour.

TABLE 1

| The coefficients of molar extinction of the products referred to in examples 1-4 are shown in logarithmic form in table 1. The measurements were made in a solution of DMF | | |
|---|---|---|
| PRODUCT | 1 max | log |
| 1 | 465 | 4.172 |
| 2 | 450 | 4.249 |
| 3 | 525 | 4.476 |
| 4 | 510 | 4.487 |

## Claims

1) 1,4-diketone pyrrole-[3,4-c-]-pyrroles, useful as pigments for organic polymeric materials or for the preparation of paints and varnishes, according to the general formula

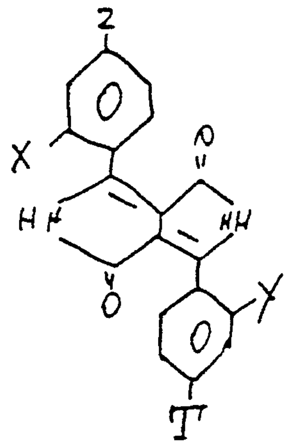

(I)

in which X and Y, equal or different from each other, are selected from among the following:
a) halogen atoms;
b) linear or branched alkylic groups, simple or substituting, containing up to five carbon atoms;
c) $-OR_3$ groups, in which $R_3$ may be an alkylic radical, either simple or substituted;
d) $-SR_3$ groups;
g) carboxylic groups;
h) ether groups;
i) amidic groups,
in which, still, Z and T, either equal or different, may have one of the meanings attributed to X or Y, or be equal to H.

2) Procedure for the preparation of 1,4-diketone pyrrole-[3,4-c]-pyrroles according to the first claim consisting in causing a benzonitrile containing appropriate substituting materials to react with an alkylic ester of succinic acid, in organic solvent and in the presence of a strong base.

3) Procedure for the preparation of 1,4-diketone pyrrole-[3,4-c]-pyrroles according to the preceding claim characterised by the fact that the reaction is carried out at a temperature of between 80 and 120 °C.

4) Procedure for the preparation of 1,4-diketone pyrrole-[3,4-c]pyrroles according to claim 2 characterised by the fact that the reaction is carried out in the presence of an organic solvent preferably selected from among tertiary alcohols.

5) Procedure for the preparation of 1,4-diketone pyrrole-[3,4-c]-pyrroles according to claim 2 characterised by the fact that the product of the reaction between the benzonitrile and the ester of the succinate acid is hydrolysed with an organic acid.

6) Method for the pigmentation of organic materials of high molecular weight consisting of incorporating in such materials effective quantities of 1,4-diketone pyrrole-[3,4-c]pyrroles according to the general formula referred to in the first claim.

7) Method for the preparation of paints and varnishes consisting of adding to the basic composition a pyrrole-pyrrolic compound selected from among those according to the general formula referred to in the first claim.

European Patent Office

# EUROPEAN SEARCH REPORT

Application Number

EP 90 20 2158

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A | EP-A-302018 (CIBA-GEIGY_AG)<br>* the whole document *<br>--- | 1, 6 | C07D487/04<br>C08K5/3415<br>C09B57/00<br>//(C07D487/04, 209:00,209:00) |
| A<br><br>D | EP-A-184982 (CIBA-GEIGY AG)<br>* the whole document *<br>& US-A-4659775<br>--- | 1, 6 | |
| A | DE-A-3713459 (LANGHALS,HEINZ,PROF.DR.)<br>* the whole document *<br>----- | 1, 6 | |
| | | | TECHNICAL FIELDS SEARCHED (Int. Cl.5 ) |
| | | | C07D487/00<br>C09B57/00<br>C08K |

The present search report has been drawn up for all claims

| Place of search | Date of completion of the search | Examiner |
|---|---|---|
| BERLIN | 12 DECEMBER 1990 | KYRIAKAKOU, G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document